# FASCICULE DE BREVET EUROPEEN

(11) **EP 3 980 014 B1**
(45) Date de publication et mention de la délivrance du brevet: **09.08.2023**
(21) Numéro de dépôt: 20729784.7
(22) Date de dépôt: 05.06.2020
(51) Int. Cl.: A61K 31/4741, A61P 27/02, A61K 31/728, A61K 31/78

(54) **UTILISATION D'UNE MOLECULE OPIOÏDE POUR TRAITER L'OEIL SEC ET L'OEIL ALLERGIQUE**
VERWENDUNG EINES OPIOID-MOLEKÜLS ZUR BEHANDLUNG VON TROCKENEM AUGE UND AUGEN, DIE AN ALLERGIEN LEIDEN
USE OF AN OPIOID MOLECULE FOR TREATING DRY EYE AND EYES SUFFERING FROM ALLERGIES

(30) Priorité: 07.06.2019 FR 1906077
(43) Date de publication de la demande: 13.04.2022
(73) Titulaire: H4 Orphan Pharma, 21000 Dijon (FR); Terrasse, Gaëtan, 21000 Dijon (FR)
(72) Inventeur: BUR, Catherine, 21000 Dijon (FR); TERRASSE, Gaëtan, 21000 Dijon (FR)
(74) Mandataire: Oudin, Stéphane
(86) Numéro de dépôt international: PCT/EP2020/065610
(87) Numéro de publication internationale: WO 2020/245345

(56) Documents cités:
- WO-A2-2007/117704
- US-A1- 2012 053 199

## Description

### Domaine technique de l'invention

La présente invention se rapporte à l'utilisation de substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one pour traiter l'œil sec et l'œil allergique.

La présente invention concerne l'utilisation d'une molécule opioïde ayant une activité sur le modulateur du régulateur de conductance transmembranaire (CFTR) et sur la modulation de l'expression du CD63 pour le traitement de l'œil sec, et de l'œil allergique ainsi que des compositions pharmaceutiques, et des procédés de traitement.

### Etat de la technique

La sécheresse oculaire ou syndrome de l'œil sec est définie par le Dry Eye Workshop de 2007 comme une maladie multifactorielle des larmes et de la surface oculaire entraînant des symptômes d'inconfort, une perturbation visuelle et une instabilité du film lacrymal avec des lésions potentielles de la surface oculaire. Elle est accompagnée d'une augmentation de l'osmolarité du film lacrymal et d'une inflammation de la surface oculaire. Les étiologies sont multiples, et on distingue classiquement les syndromes secs par diminution du flux lacrymal (comme le syndrome de Gougerot-Sjögren) et des syndromes secs par hyper évaporation des larmes.

Le dysfonctionnement des glandes de Meibomius (MGD) est la cause la plus fréquente de maladie de la sécheresse oculaire (Dry Eye Disease en anglais ou DED). Un workshop de 2011 à défini également ce qu'est Le dysfonctionnement des glandes de Meibomius. L'inflammation des paupières, la croissance microbienne, les troubles cutanés associés ainsi que les complications potentiellement graves de la cornée aboutissent à faire de la MGD un trouble multifactoriel complexe. Il est probable que la MGD soit une affection hétérogène résultant de l'association des cinq mécanismes physiopathologiques distincts suivants : inflammation des paupières, inflammation de la conjonctive, lésions de la cornée, modifications microbiologiques et DED résultant de l'instabilité du film lacrymal. La pathogénie à la fois du MGD et du DED peut être décrite en termes de « cercle vicieux » : les mécanismes physiopathologiques sous-jacents du DED et du MGD interagissent, aboutissant à un double cercle vicieux. C'est ce double cercle vicieux qui est à l'origine de l'œil sec et de sa difficulté à le traiter.

La sécheresse oculaire (Dry Eye Disease en anglais ou DED) est une maladie multifactorielle et complexe de la surface oculaire qui entraîne une perte de l'homéostasie du film lacrymal et entraîne des symptômes oculaires variables. L'effet négatif de la DED sur la fonction visuelle, la qualité de vie et le fardeau économique est bien reconnu. (Efficacy of topical ophthalmic drugs in the treatment of dry eye disease: A systematic literature review. Holland EJ, et coll Ocul Surf. 2019*).* Chez de nombreux patients, la maladie est chronique et nécessite un traitement à long terme.

La prévalence du DED est élevée, avec des estimations mondiales allant de 5 à 50 % de la population adulte, et le fardeau économique de la maladie devrait augmenter avec l'âge de la population. Dans le monde entier, les lubrifiants oculaires sont souvent utilisés lors de la prise en charge initiale du DED, mais n'abordent pas les causes sous-jacentes de la maladie. Des médicaments pharmacologiques ophtalmiques potentiellement plus efficaces, ciblant différentes voies physiopathologiques distinctes du DED, ont été étudiés au cours des deux dernières décennies, mais ces efforts ont abouti à l'approbation de très peu de nouveaux médicaments. Les principaux traitements approuvés sont l'émulsion ophtalmique à la cyclosporine A à 0,05 % (Restasis^{®} ; Allergan, Irvine, Californie, USA) et la solution ophtalmique lifitegrast à 5,0% (Xiidra^{®}; Shire, Lexington, États-Unis) en Amérique du Nord, émulsion cationique. ; Ikervis^{®} ; Santen Pharmaceutical, Osaka, Japon) en Europe et solution ophtalmique à base de diquafosol à 3 % (Diquas ^{®} ; Santen Pharmaceutical, Osaka, Japon) et une dose unitaire de suspension ophtalmique à 2 % de rebamipide (Mucosta^{®} ; Otsuka Pharmaceutical, Tokyo, Japon) en Asie. Aux États-Unis (août 2018), une formulation nano micellaire de Cyclosporine A à 0,09 % (Cequa ^{™} ; Sun Pharmaceuticals, Mumbai, Inde) a été approuvée pour augmenter la production de larmes chez les patients atteints de DED. Globalement, ces médicaments réduisent l'inflammation de la surface oculaire ou stabilisent le film lacrymal, bien que l'on ne sache pas quels médicaments conviennent le mieux aux patients présentant un DED déficient en solution aqueuse (ADDE) ou un DED évaporatif. Le syndrome de Gougerot-Sjögren (SGS) est associé à une xérophtalmie due à la destruction progressive des glandes lacrymales, pouvant être responsable d'une kératite sévère. Le SGS est une affection chronique d'origine auto-immune caractérisée par une atteinte inflammatoire, progressive et dégénérative des glandes exocrines, à laquelle peut être associée une pathologie systémique touchant de façon variable les articulations, la peau, les poumons, les reins ou les nerfs périphériques. La physiopathologie de la maladie est caractérisée par une infiltration des glandes salivaires et lacrymales par des lymphocytes T CD4+ et par des lymphocytes B. L'activation locale et la prolifération de ces lymphocytes déclenchent la libération de cytokines pro inflammatoires qui entretiennent un état d'inflammation chronique, ainsi que la sécrétion d'autoanticorps, conduisant à terme à la mort par apoptose des cellules épithéliales.

Les conjonctivites allergiques saisonnières et pérennes (allergies oculaires) sont caractérisées par des yeux irritants, rouges, gonflés et larmoyants. Les symptômes aigus de la conjonctivite allergique se caractérisent par les signes cliniques et les symptômes de démangeaisons oculaires, de rougeurs et d'enflures. Les réactions inflammatoires de la conjonctivite allergique en phase tardive ou d'allergie incluent la rougeur, le gonflement de la paupière et des larmoiements. La conjonctivite allergique et la rhinoconjonctivite peuvent également coexister avec d'autres affections oculaires externes et des maladies telles que la sécheresse oculaire ou des irritations causées par des polluants ou d'autres causes. Cela conduit à un film lacrymal compromis, qui sert à protéger la surface oculaire des allergènes. L'allergie oculaire est une cause importante d'œil sec. De nombreux traitements existent pour traiter la conjonctivite allergique, mais aucun pour traiter l'œil sec provoqué par une pathologie allergique.

La prise en charge usuelle et initiale du syndrome de l'œil sec, quelle que soit l'étiologie repose sur :
- la correction des facteurs favorisants, autant que possible (médicaments, facteurs environnementaux, collyres contenant des conservateurs, en particulier les ammoniums quaternaires ;
- et un traitement substitutif par substituts lacrymaux [larmes artificielles en collyres, gels, ainsi que les dispositifs médicaux de solutions viscoélastiques utilisés après échec des 2 autres].

Une fois amorcée, la sécheresse oculaire peut évoluer malgré un traitement substitutif et s'auto entretenir selon le concept du cercle vicieux de l'inflammation avec progressivement une atteinte de l'ensemble des tissus la surface oculaire, dont la cornée. Dans les formes sévères, la sécheresse oculaire peut être responsable d'une atteinte majeure de la cornée [ou kératite] qui entraine un cortège de symptômes allant d'une sensation de corps étranger à la surface oculaire et de brûlure jusqu'à une douleur permanente avec baisse de l'acuité visuelle. La gravité de la sécheresse oculaire est liée à l'importance de la kératite, de la composante inflammatoire et des symptômes oculaires.

Il est nécessaire de traiter plusieurs cibles pharmacologiques pour traiter les causes des cercles vicieux responsables de l'œil sec. Ainsi il est nécessaire de relancer la production de larmes émises par les glandes lacrymales, mais également la sécrétion d'électrolytes par l'épithélium cornéen.

Il a été démontré dans un article publié en 2001 que le CFTR avait un rôle dans la sécrétion des électrolytes sur une lignée de cellules épithéliales cornéennes de lapin immortalisé (Invest Ophthalmol Vis Sci. 2001 Sep Activation of a CFTR-mediated chloride current in a rabbit corneal epithelial cell line. Al-Nakkash L). Mais également sur la sécrétion des glandes lacrymales (Invest Ophthalmol Vis Sci. 2018 Jan Novel Insight Into the Role of CFTR in Lacrimal Gland Duct Function in Mice. Berczeli O.). Dans cet article les auteurs montrent qu'un modulateur du CFTR est capable d'augmenter la sécrétion des larmes chez des souris possédant une CFTR normal.

Le CFTR est un canal anionique médié par l'AMPc /ATP qui est exprimé dans une variété de types de cellules, y compris les cellules épithéliales sécrétoires, où il régule le flux anionique à travers la membrane, ainsi que l'activité d'autres canaux ioniques et protéines. Dans les cellules épithéliales, le fonctionnement normal du CFTR est essentiel pour le maintien du transport des électrolytes dans tout le corps, y compris les tissus respiratoires et digestifs et même oculaires. Le CFTR est composé d'environ 1480 acides aminés constituant une protéine composée d'une répétition de domaines transmembranaires, chacun contenant six hélices transmembranaires et un domaine de liaison de nucléotide. Les deux domaines transmembranaires sont reliés par un grand domaine polaire régulateur [R] avec plusieurs sites de phosphorylation qui régulent l'activité des canaux et le trafic cellulaire.

Le transport du chlorure se produit par l'activité coordonnée de l'ENaC et du CFTR présents sur la membrane apicale et des canaux Na+ et K+ ATPase et Cl - exprimés sur la surface basolatérale de la cellule. Le transport actif secondaire du chlorure du côté de la lumière entraîne l'accumulation de chlorure intracellulaire, qui peut ensuite quitter la cellule de manière passive par les canaux Cl⁻, ce qui entraîne un transport du pôle basal au pôle apical. Ainsi l'eau qui n'est probablement jamais activement transportée, est transportée à travers les épithéliums selon des gradients osmotiques transépithéliaux générés par le flux du sodium et des chlorures.

Il y a une nécessité de trouver de nouveaux traitements pour moduler et activer le fonctionnement du CFTR normal pour traiter la cause de l'œil sec qui semble impacter par un fonctionnement insuffisant du CFTR.

La conjonctivite allergique est l'une des affections allergiques les plus courantes dans le monde. Son incidence augmente en raison des changements climatiques, de la pollution, de l'augmentation de la charge de pollen et de la sensibilité immunologique accrue du sujet en réponse à ces changements environnementaux. La physiopathologie implique principalement l'activation des mastocytes liée à l'immunoglobuline E, la libération d'histamine et d'autres médiateurs contribuant à la propagation de la réponse en faisant appel à d'autres cellules immunitaires et à une inflammation ultérieure.

La conjonctivite allergique (CA) a une prévalence croissante dans le monde entier. Les démangeaisons oculaires sont le symptôme pathognomonique de l'AC, ce qui lui permet de poser un diagnostic différentiel par rapport à d'autres affections oculaires résultant d'irritations non allergiques.

La conjonctivite allergique saisonnière (CAS) et la conjonctivite allergique pérenne (CAP) sont les formes les plus courantes de sous-groupes d'allergies oculaires et on estime qu'elles affectent 15 à 25% de la population américaine ( Ono et coll, 2005, Allergie conjunctivitis: update on pathophysiology and prospects for future treatment. J Allergy Clin Immunol).En Europe, la conjonctivite allergique est en hausse, touchant jusqu'à 50% de la population, probablement en raison de l'introduction de l'ambroisie (Burbach G., et coll. 2009, Ragweed sensitization in Europe LEN study suggests increasing prevalence. Allergy). Les patients atteints de CA présentent généralement des démangeaisons, un larmoiement, une sensation de brûlure, une vasodilatation et un chemosis de façon bilatéral. La kératoconjonctivite printanière (KCP), la keratoconjonctivite chronique, morbide est potentiellement menaçante pour la vision. Ces différentes pathologies allergiques chroniques sont accompagnées souvent d'un syndrome de l'œil sec (Villani et collaborateurs, ocular allergy dry eye, Curr Opin All CLin Immunol, 2018).

De nombreux traitements sont utilisés, ce sont les collyres antihistaminiques, les collyres à base de cortisone et également des collyres à base d'immunosuppresseurs, telle la cyclosporine.

WO 2007/117704 divulgue l'utilisation de la tritoqualine dans le traitement de l'allergie et plus particulièrement des conjonctivites allergiques.

Aucun traitement ne traite spécifiquement l'allergie oculaire et ses conséquences comme l'œil sec.

Il est urgent de trouver des solutions pour traiter à la fois l'inflammation de l'œil allergique mais également ses conséquences.

Il y a une nécessité de trouver de nouveaux traitements pour moduler et activer le fonctionnement du CFTR mais également d'empêcher la dégranulation du mastocyte pour traiter la cause intrinsèque de la maladie.

### Présentation de l'invention

La présente invention concerne des composés, comprenant des composés opioïdes et leurs sels pharmaceutiquement acceptables.

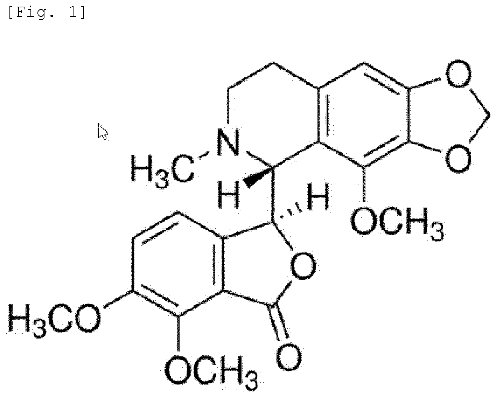

Les inventeurs ont découvert les propriétés étonnantes du (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one, ou noscapine (cf [Fig.1]) sur la modulation du CFTR.

L'invention concerne également des compositions pharmaceutiques comprenant au moins l'un des composés décrits dans le présent document et/ou au moins un de leurs sels pharmaceutiquement acceptables, ces compositions pouvant comprendre en outre au moins un autre ingrédient pharmaceutique actif et/ou au moins un excipient.

L'invention concerne des procédés de traitement de l'œil sec, de la conjonctivite allergique ou de la maladie de Gougerot-Sjögren, consistant à administrer au moins l'un des composés décrits dans le présent document et/ou au moins un de ses sels pharmaceutiquement acceptables, éventuellement en tant que partie d'une composition pharmaceutique comprenant au moins un composant supplémentaire, à un sujet en ayant besoin.

La noscapine est une substance chimique connue depuis de très nombreuses années, et utilisée comme antitussif. La noscapine est un alcaloïde non narcotique de phtalide isoquinoléine dérivé du pavot à opium, Papaver somniferum, doté d'activités analgésiques, antitussives et antinéoplasiques légères. La noscapine exerce ses effets antitussifs en activant les récepteurs sigma opioïdes. Cet agent semble exercer son effet antimitotique en se liant à la tubuline, ce qui perturbe la dynamique d'assemblage des microtubules et, par la suite, inhibe la mitose et la mort des cellules tumorales.

Les doses thérapeutiquement efficaces répondent essentiellement aux effets indésirables de codéine et, sauf pour les nausées occasionnelles, ses effets indésirables sont négligeables. Les doses jusqu'à 90 mg par jour n'ont aucun effet sur la respiration chez l'homme.

La noscapine est un alcaloïde benzylisoquinoléine substituée par un groupe 4,5-diméthoxy-3-oxo-1,3-dihydro-2-benzofuranne-1-yle position 1, un groupe méthylènedioxy en positions 6 et 7 et un groupe méthoxy en position 8. Obtenu à partir de plantes de la famille des papavéracées, elle n'a pas de propriétés antidouleur significatives et est principalement utilisée pour ses effets antitussifs. La noscapine, de formule brute C22H23NO7, à un poids moléculaire de 413. Ce composé peut être modifié ou substitué par des composés comprenant soit du carbone 14 ou des composés deutériés.

Les composés et les sels marqués par un isotope peuvent être utilisés de différentes manières. Ils peuvent convenir à des médicaments et/ou à différents types de tests, tels que des tests de distribution de tissu sur substrat. Par exemple, les composés marqués au tritium et/ou au carbone 14 sont particulièrement utiles pour divers types de tests, tels que les tests de distribution tissulaire sur substrat, en raison de leur préparation relativement simple et de leur excellente détectabilité. Par exemple, les produits marqués au deutérium sont utiles sur le plan thérapeutique et présentent des avantages thérapeutiques potentiels par rapport aux composés non marqués par le deutérium. En général, les composés et les sels marqués au deutérium peuvent avoir une stabilité métabolique supérieure à ceux qui ne sont pas marqués par des isotopes en raison de l'effet cinétique isotopique. Une stabilité métabolique plus élevée se traduit directement par une demi-vie accrue in vivo ou par des doses plus faibles, ce qui pourrait être souhaité. Les composés et les sels marqués par un isotope peuvent généralement être préparés en suivant les procédures décrites dans les schémas de synthèse connues. Les descriptions associées dans la littérature mondiale comme par exemple le brevet EP3352757. Il est ainsi aisé de remplacer des méthyls non deutériés par des méthyls deutériés. La noscapine se présente sous la forme d'une poudre blanche cristalline. Insoluble dans l'eau; soluble dans le benzène et l'acétone. Les sels formés avec des acides sont dextrorotatoires.

Les inventeurs ont démontré que la noscapine avait une action très importante sur le CFTR mais également sur l'inhibition de la dégranulation du basophile, qui est le pendant cellulaire du mastocyte. Ces deux actions combinées sont majeures pour traiter l'œil allergique et l'œil sec.

De nombreux brevets ont été déposés sur la noscapine, mais aucun ne parle de son activité sur l'œil sec ou l'œil allergique.

### Brève description des figures

[Fig. 1] représente la structure chimique de la noscapine.
[Fig. 2] Activité de la noscapine au pôle apical exprimé en pourcentage de l'effet maximal.
[Fig. 3] Activité de la noscapine au pôle basal exprimé en pourcentage de l'effet maximal.

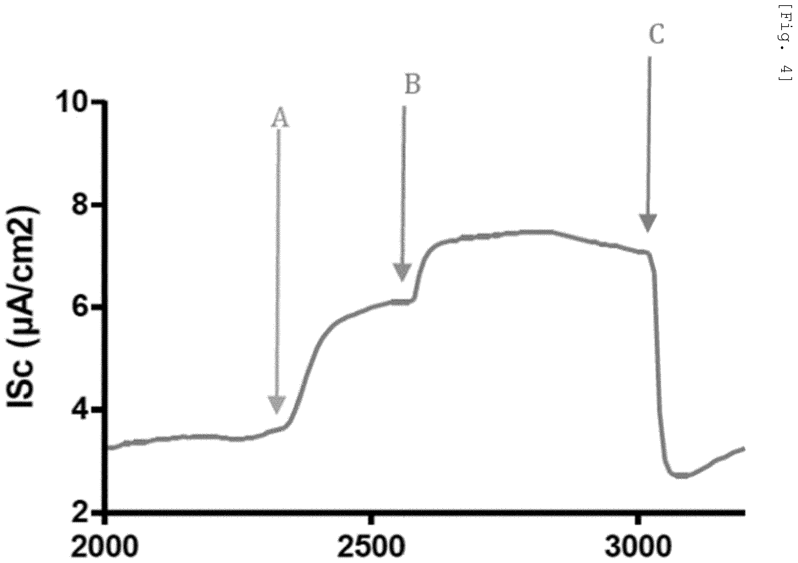

[Fig. 4] Effet de la noscapine exprimé en µA/Cm² au pôle apical sur cellules épithéliales de la glande lacrymale (À= Forskoline ;B= Noscapine ;C= Inh 172).
[Fig. 5] Effet de la noscapine exprimé en µA/Cm² au pôle basal sur cellules épithéliales de la glande lacrymale (À= Forskoline ;B= Noscapine ;C= Inh 172).

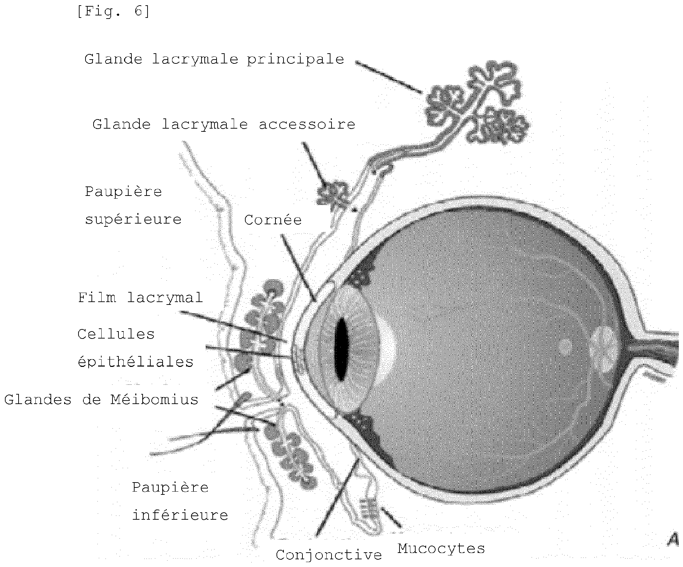

[Fig. 6] l'unité fonctionnelle lacrymale d'après DARTT 2002.
[Fig. 7] témoin positif, analyse par cytométrie de flux mettant en évidence la dégranulation du basophile par les anticorps anti-FcεRI, qui est l'anti-récepteur des IgE (CD63+et CCR3+).
[Fig. 8] témoin négatif en cytométrie de flux
[Fig. 9] Inhibition de la dégranulation du basophile par la noscapine (10 µM).

Les inventeurs ont mis en évidence les propriétés étonnantes et surprenantes de la noscapine dans un modèle cellulaire humain de modulation du CFTR sans mutation.

Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement des maladies associées à la baisse des sécrétions des cellules épithéliales du tissu conjonctival, des glandes lacrymales et des glandes de Meibomius.

La présente invention concerne également, les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement des maladies associés à l'allergie oculaire. Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec.

Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec lié à la maladie de Gougerot-Sjögren et des syndromes Gougerot-Sjögren secondaires qui accompagnent des maladies auto-immune tel que la polyarthrite rhumatoïde ou le lupus érythémateux disséminé.

Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement des conjonctivites allergiques avec baisse de sécrétion du film lacrymal.

Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la keratoconjonctivite vernale.

Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec caractérisée en ce qu'elle est administrée sous la forme de collyre ou de pommade oculaire.

Ainsi la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec et/ou la maladie de l'œil allergique caractérisée en ce qu'elle est administrée sous la forme de collyre à la dose de 0.1 milligramme à 5 milligrammes.

Selon un mode de réalisation préféré, la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec caractérisée en ce qu'elle est administrée sous la forme de collyre en association avec des humectants et des lubrifiants à base d'acide hyaluronique ou de carbomères. Ces composés ont la capacité d'augmenter la rémanence de l'effet de la noscapine. Selon un mode de réalisation préféré, la présente invention se rapporte à des substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec et/ou la maladie l'œil allergique caractérisée en ce qu'elle est substituée au niveau d'au moins un de ses méthyls par des méthyls deutériés. La présente invention concerne également les substances chimiques : les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans l'amélioration du transport ionique aussi bien au niveau apical qu'au pôle basal de la cellule épithéliale cornéenne, la cellule épithéliale palpébrale, glandulaire lacrymale et/ou épithéliales glandulaires de Meibomius.

Selon le mode préféré de l'invention, ladite substance chimique est substituée au niveau de ses méthyls par des méthyls deutériés pour améliorer la pharmacocinétique.

Selon un mode de réalisation préféré, l'invention concerne les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de la maladie de l'œil sec à la dose de 0.1 milligramme à 5 milligramme/jour.

Selon un mode de réalisation préféré, l'invention concerne les énantiomères lévogyres et dextrogyres de la (3S) -6,7-diméthoxy-3 - [(5R) -4-méthoxy-6-méthyl-7,8-dihydro-5H- [1,3] dioxolo [4,5-g] isoquinoline-5-yl] -3H-2-benzofuranne-1-one et ses sels pharmaceutiquement acceptables pour utilisation dans le traitement de l'œil sec remarquable en ce qu'elle est conditionnée sous forme de collyre, ou pommade oculaire.

### Exemples

Pour étudier cette action sur la modulation du CFTR les inventeurs ont utilisé la méthode des chambres d'Ussing, inventées par le Danois Hans H. Ussing à la fin des années cinquante. Cette technique permet d'étudier les échanges ioniques à travers un épithélium, car elles permettent de maintenir en survie pendant quelques heures le tissu d'intérêt dans des conditions contrôlées de température et de milieu. Le positionnement du tissu entre deux demi-chambres permet de définir un compartiment apical (correspondant à la lumière de l'organe) et un compartiment basolatéral (correspondant au compartiment sanguin) et d'étudier les échanges entre ces deux compartiments via le tissu.

Cette technique est utilisée en routine et elle est particulièrement bien adaptée pour une approche pharmacologique des transports ioniques et pour rechercher des molécules d'intérêt thérapeutique dans le cadre des sécrétions ioniques des cellules épithéliales. Elle consiste en la mesure du courant transépithélial (dit de court-circuit et noté Ise). Ise est exprimé en ampère rapporté à l'unité de surface de l'épithélium (Ise en µA/cm²). Les cellules sont cultivées sur filtre poreux pendant 10 à 15 jours à l'interface liquide-liquide puis air-liquide afin de mimer des conditions proches de *l'in vivo.* La résistance transépithéliale est mesurée régulièrement au cours de la culture. Plus cette résistance est élevée (plusieurs centaines d'ohms) plus le tissu épithélial est jointif, polarisé et donc étanche. À l'interface Air (côté apical) - Liquide (côté basolatéral) les cellules épithéliales se polarisent et forment un tapis étanche qui peut être étudié avec la technique de la chambre d'Ussing. Nous avons utilisé un système avec 6 cuves de chez Physiologie Instrument^{®} permettant 6 expériences en parallèle.

Nous avons utilisé le matériel et les molécules suivantes dans notre étude : Amiloride : 100 µM concentration finale ; solution mère à 100mM, solvant eau (fournisseur Sigma^{®}). Forskoline : 0,05 µM concentration finale ; solution mère à 1 mM, solvant DMSO (fournisseur Sigma^{®}). Genisteine : 30 µM concentration finale ; solution mère à 30mM, solvant DMSO (fournisseur Sigma^{®}). CFTR inh172 : 10 µM concentration finale ; solution mère à 10mM, solvant DMSO (fournisseur Fisher^{®}). UTP : 100 µM concentration finale ; solution mère à 100 mM, solvant DMSO (fournisseur Sigma^{®}).

Nous avons utilisé divers milieux et réactifs dont des supports adaptés à la chambre d'Ussing : Snapwell (Fisher ^{®}), milieu de culture (Gibco ^{®}),SVF (Gibco^{®}), Puromycine (Gibco ^{®}), Flacons de culture T75 (Fisher^{®}).

Les inventeurs ont utilisé des chambres d'USSING pour analyser l'activité de la noscapine sur le CFTR de cellules épithéliales glandulaires.

Protocoles types utilisés pour les cellules épithéliales humaines exprimant le CFTR non muté :
- Mesure du courant de court-circuit en présence d'amiloride (inhibiteur du canal ENaC, 100 µM) puis ajout de 10 µM de la molécule noscapine puis ajout de CFTR inh172 (10 µM, inhibiteur de CFTR) puis ajout d'UTP (100 µM, valide l'expérience en activant le transport Cl calcium sensible).
- Mesure du courant de court-circuit en présence d'amiloride (100 µM) et de forskoline (0,05 µM activateur de l'AMPc intracellulaire) puis ajout de 10 µM de la molécule noscapine puis ajout de CFTRinh172 (10 µM) puis ajout d'UTP (100 µM).
- Mesure du courant de court-circuit en présence CFTR Inh172 (10 µM) puis ajout de la noscapine et de forskoline (0,05 µM) puis ajout d'UTP.

Les inventeurs ont préparé une solution mère à 100 µM dans du DMSO. Les composés ont été aliquotés par 100 µL et stockés à -20° C.

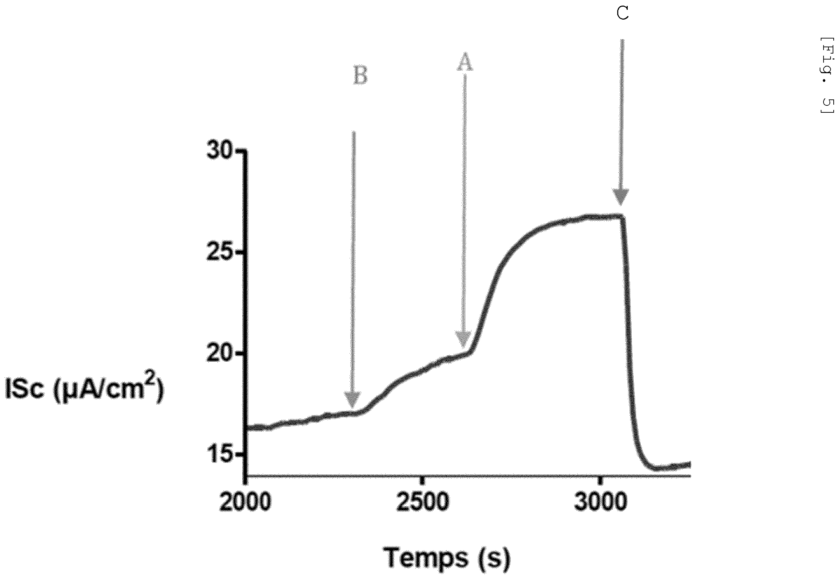

La noscapine a été ajoutée dans les cellules d'USSING à la dose de 10 µM sur des cellules non mutées [Fig. 5].

L'effet de la noscapine sur cellules épithéliales non mutées coté apical est important, car le différentiel passe de 16 à plus de 20 (exprimé en µA/cm²).

L'ajout de forskoline modifie le potentiel qui passe de 20 à 27 (exprimé en µA/cm²).

L'ajout de l'Inh172 bloque complètement le potentiel cellulaire.

La noscapine active le potentiel cellulaire dans les cellules épithéliales des glandes lacrymales exprimant le CFTR non muté. Cet effet est additif avec celui de la forskoline.

L'effet de la noscapine sur cellules épithéliales des glandes lacrymales non mutées coté apical est important, car le différentiel passe de 6.5 à plus de 7.5 (exprimé en µA/cm²).

L'ajout de forskoline modifie le potentiel qui passe de 3.5 à 6.5 (exprimé en µA/cm²).

L'ajout de l'Inh172 bloque complètement le potentiel cellulaire.

En conclusion la noscapine active, dans les cellules épithéliales de la glande lacrymale, le transport ionique.

Cet effet est bloqué par l'inh172, qui inhibe spécifiquement le transport ionique du CFTR.

La noscapine apparait comme une molécule apte à stimuler le transport ionique via le CFTR non muté.

Nous avons ensuite déterminé la dose efficace de la noscapine à la fois sur le pôle apical et le pôle basal.

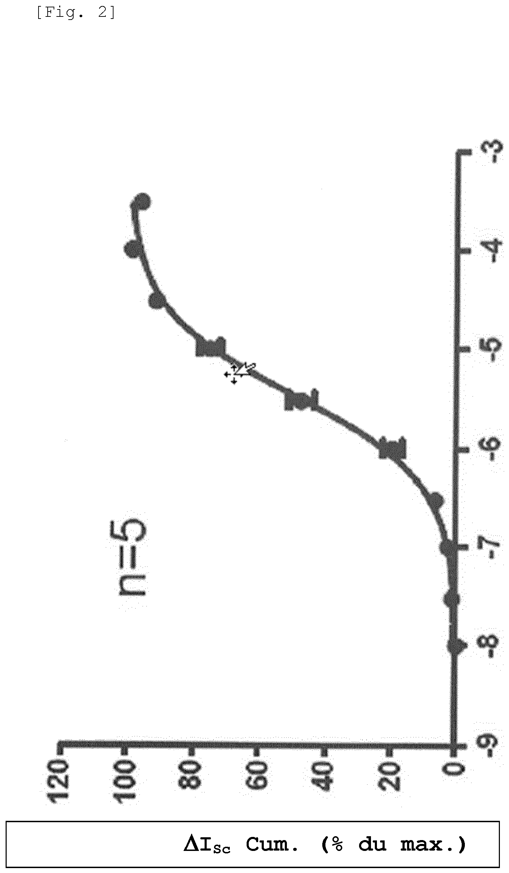

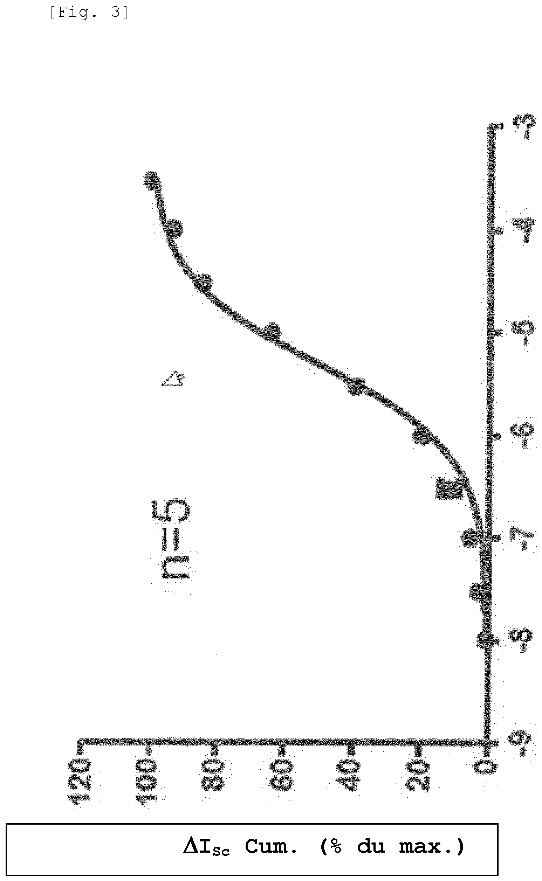

Le résultat donne une activité de l'EC50 de 3.42 +/- 0.19 µM pour le pôle apical et une EC50 de 4.87 +/- 0.27 µM pour le pôle basal [Fig. 2], [Fig.3].

Ainsi la noscapine permet d'améliorer la fonction de sécrétion lacrymale des patients ayant une pathologie avérée d'œil sec y compris des patients ayant une maladie de Gougerot - Sjögren, mais également des Gougerot - Sjögren dits « secondaires » car ils sont associés à des maladies autoimmunes spécifiques comme la polyarthrite rhumatoïde ou le lupus érythémateux disséminé.

Pour démontrer l'effet de la noscapine sur l'œil allergique les inventeurs ont travaillé sur un modèle cellulaire d'allergie, le basophile. Dans la muqueuse conjonctivale on trouve des mastocytes mais également des basophiles. Les mastocytes et les polynucléaires basophiles humains sont des éléments d'aspect morphologique relativement similaire, dérivant de la même cellule souche hématopoïétique CD34+.

Tandis que les mastocytes sont des éléments résidant dans les tissus, le basophile est une cellule circulante, mais en cas de poussée allergique importante peut se retrouver dans les tissus épithéliaux (Kepley and coll, J Respir Crit Gare Med. 2001. Immunohistochemical détection of human basophils in post mortem cases of fatal asthma).

Ces deux cellules interviennent dans la réaction allergique IgE-dépendante ; elles expriment le récepteur de haute affinité des IgE. Néanmoins, les médiateurs libérés par ces cellules au cours de cette activation sont pour certains différents. Par ailleurs, basophiles et surtout mastocytes interviennent dans l'immunité innée. Ainsi, les basophiles et les mastocytes ont de nombreuses caractéristiques communes et plus particulièrement, la sécrétion d'IL4 et d'IL13 qui sont réputées pour induire la stimulation de synthèse de collagène et la prolifération de fibroblastes responsables de fibroses. La fibrose peut atteindre les glandes de Meibomius dans l'œil sec et l'œil allergique.Les basophiles sont présents dans le sang circulant contrairement aux mastocytes dont ils présentent la majorité des propriétés. Pour étudier l'action de la noscapine sur le basophile, nous avons utilisé un kit commercial, le kit Flow CAST^{®} de BULHMANN Laboratories AG (Suisse) qui est un test d'activation des basophiles (TAB) pouvant être utilisé pour la détection *in vitro* de la dégranulation des basophiles ainsi que pour l'étude des réactions allergiques de type immédiates et les hypersensibilités.

Il est conçu pour le diagnostic *in vitro* de l'expression du marqueur CD63 comme marqueur de surface des basophiles activés. Le test est réalisé sur sang total; la cytométrie en flux permet de quantifier l'expression du CD63 à la surface des basophiles activés (www.buhlmannlabs.ch).

Les inventeurs ont utilisé Le Kit-Flow CAST (www.buhlmannlabs.ch/products-solutions/cellular-allergy/flow-cast/) pour tester l'action de la noscapine sur l'inhibition de la dégranulation du basophile. L'activation (ou dégranulation) des basophiles peut se faire de trois façons différentes soit par un allergène, ou bien par un « anti IgE » (anti-FcεRI, qui est l'anti-récepteur des IgE) ou encore par un antigène lipopolysaccharidique bactérien, appelé le fMLP. Les basophiles au repos n'expriment que très peu l'antigène CD63 car il est lié aux granules intracytoplasmiques.

L'activation des basophiles (par exemple par une IgE (activation immunologique) ou le fMLP (activation non immunologique)) entraîne la fusion des granules avec la membrane plasmique et donc l'expression du CD63 à la surface des cellules.

Pour évaluer la dégranulation, le kit Flow CAST^{®} a été utilisé partiellement. Ce test comprend un anti IgE du récepteur à l'IgE.

Le CCR3 est une protéine codée par un gène qui est un récepteur pour les chimiokines de type C-C. Il appartient à la famille 1 des récepteurs couplés à la protéine G. Il est fortement exprimé dans les éosinophiles, dans les basophiles et est également détecté dans les cellules TH1 et TH2, ainsi que dans les cellules épithéliales.

La cytométrie de flux permet de caractériser les différentes cellules sanguines. Les faisceaux lasers permettent l'évaluation et la mesure de différents paramètres cellulaires. La mesure frontale de la lumière diffractée du faisceau laser permet d'évaluer la taille de la cellule : c'est le Forward SCatter (FSC). La mesure de la lumière diffractée perpendiculairement permet d'évaluer la granularité de la cellule : c'est le Side SCatter (SSC).

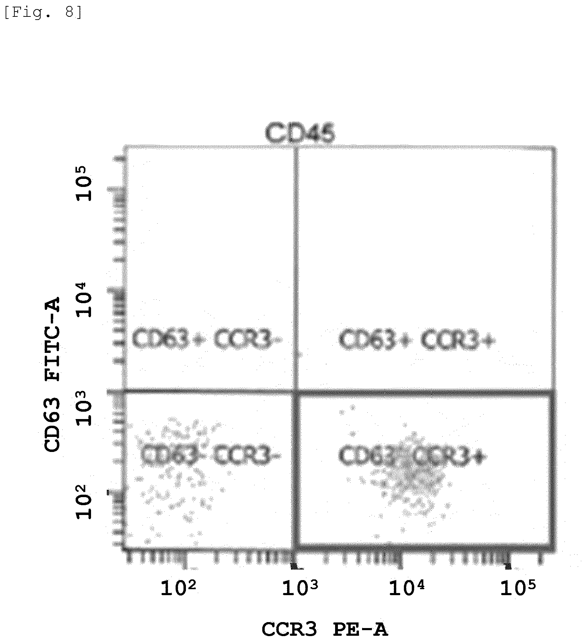

Cette granularité peut être due à des irrégularités à l'intérieur ou à la surface des cellules ou bien, à la densité des organites qui la composent (acess.ens-lyon.fr). Des marqueurs de fluorescence permettent ensuite de mieux caractériser les différentes sous populations cellulaires (ces marqueurs sont couplés avec les clusters de différentiations). Les inventeurs ont choisi un protocole de dégranulation. Ainsi 4 patients ont été choisis aléatoirement parmi des patients ayant fait l'objet d'une prise de sang classique (NF) dans un laboratoire de biologie médicale. Rappel, lors de l'activation des basophiles les marqueurs CD63 liés aux granules intra- cytoplasmiques vont fusionner avec la membrane plasmique. Ils sont alors exprimés à la surface de la cellule : les basophiles activés deviennent de ce fait CD63+. Outre le CD63, un autre marqueur spécifique des basophiles permet de mieux les cibler : il s'agit du CCR3 (récepteur de chimiokines 3). Ce dernier est toujours exprimé par ce type cellulaire. Les basophiles activés et dégranulés sont CD63+ et CCR3+ ; les basophiles non-dégranulés sont CD63- et CCR3+. Tout d'abord, un échantillon « témoin négatif », a été défini, c'est celui contenant uniquement le tampon neutre. Ce dernier a été analysé afin d'observer les résultats attendus lors de l'absence de stimulation et donc de dégranulation. Nous pouvons constater, sur la [Fig. 8], que seule la zone CD63- CCR3+ (en bleu) contient un nuage de points correspondant aux basophiles non dégranulés.

En l'absence de stimulation, les cellules ne sont donc pas activées et ne dégranulent pas. L'échantillon témoin positif est réalisé avec l'anticorps FcεRI.

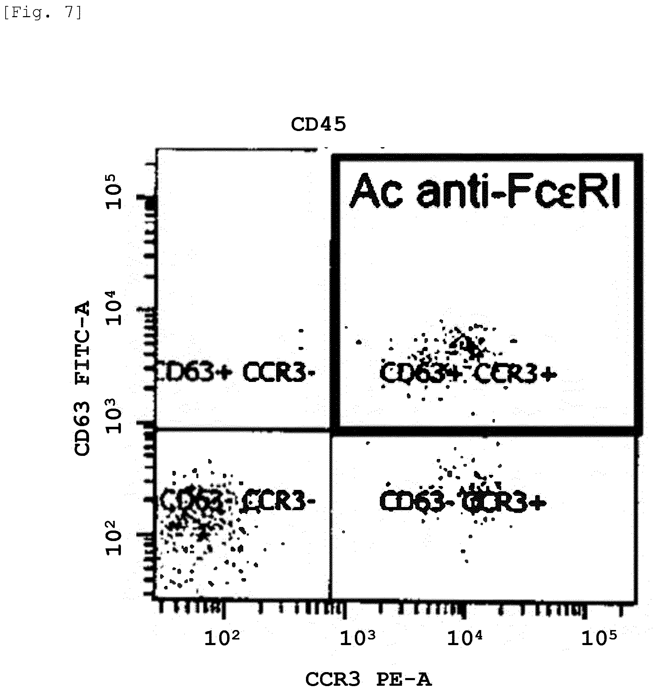

Une fenêtre (Échantillon témoin positif avec l'Anticorps anti-FcεRI) permet d'observer les nuages de points dans la zone CD63+ CCR3+ (en rouge). Le produit utilisé (Ac anti-FcεRI) entraîne bien la dégranulation des Basophiles (cf [Fig. 7]).

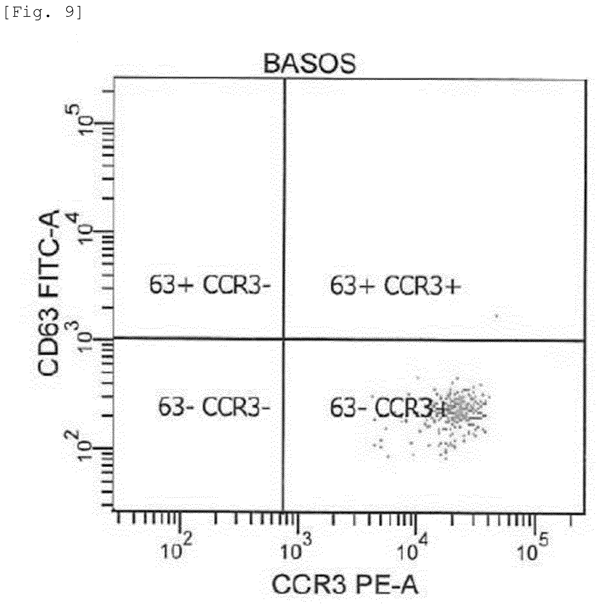

La [Fig. 9] montre que la noscapine bloque l'activation de la dégranulation du basophile provoquée par l'Anticorps anti-FcεRI.

Nous pouvons dire que la noscapine à une action surprenante sur l'inhibition de la dégranulation via la stimulation du récepteur à l'IgE.

Ainsi dans l'œil sec la noscapine aurait deux actions. Une action sur le basophile et donc le mastocyte en empêchant sa dégranulation et une autre sur le CFTR. L'action sur la dégranulation empêchant la réaction inflammatoire et l'action sur le CFTR normal permet de relancer la sécrétion des glandes lacrymales mais également des cellules épithéliales conjonctivales. Ainsi le film lacrymal qui est fortement perturbé avec une grande instabilité dans l'œil sec se retrouvera reconstitué.

Ainsi l'action locale de la noscapine sur l'œil sec apparait remarquable et étonnante par ses deux actions pharmacologiques sur le CFTR et l'inhibition de la dégranulation du basophile et du mastocyte.

## Revendications

1. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation dans le traitement des maladies associées à la baisse des sécrétions des cellules épithéliales conjonctivales, des glandes lacrymales et des glandes de Meibomius.

2. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation dans le traitement des maladies associés à l'allergie oculaire.

3. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon la revendication 1 et 2 dans le traitement de la maladie de l'œil sec.

4. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon la revendication 2 dans le traitement de la maladie de l'œil allergique lié à la keratoconjonctivite vernale.

5. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon la revendication 3 dans le traitement de la maladie de l'œil sec lié à des maladies auto-immune comme la polyarthrite rhumatoïde, le lupus érythémateux disséminé et la maladie de Gougerot - Sjögren.

6. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon la revendication 2 dans le traitement de la maladie de l'œil sec dans le traitement des conjonctivites allergiques avec baisse de sécrétion du film lacrymal.

7. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation dans le traitement de la maladie de l'œil sec selon l'une des revendications 2 à 6 **caractérisée en ce qu'**elle est administrée à la dose de 0,1 à 5 mg/jour.

8. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon l'une des revendications 1 à 7 **caractérisée en ce qu'**elle est en association avec des humectants à base de carbomères ou des lubrifiants à base d'acide hyaluronique.

9. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon l'une des revendications 1 à 8 **caractérisée en ce qu'**elle est substituée au niveau d'au moins un de ses méthyls par des méthyls deutériés.

10. Les énantiomères lévogyres et dextrogyres de la (3S) - 6,7-diméthoxy-3 - [(5R) -4-méthoxy-6-méthyl-7,8-dihydro-5H- [1,3] dioxolo [4,5-g] isoquinoline-5-yl] - 3H-2-benzofuranne-1-one et ses sels pharmaceutiquement acceptables pour utilisation selon l'une des revendications 1 à 9 **caractérisée en ce qu'**elle est conditionnée sous formes de collyre ou de pommade ophtalmique.

## Patentansprüche

1. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung bei der Behandlung von Erkrankungen, die mit einer verminderten Sekretion der Epithelzellen der Bindehaut, der Tränendrüsen und der Meibomius-Drüsen in Verbindung stehen.

2. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung bei der Behandlung von Erkrankungen, die mit Augenallergien in Verbindung stehen.

3. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach Anspruch 1 und 2 bei der Behandlung der Krankheit des trockenen Auges.

4. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach Anspruch 2 bei der Behandlung von allergischen Augenerkrankungen, die mit Keratokonjunktivitis vernalis in Verbindung stehen.

5. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach Anspruch 3 bei der Behandlung der Krankheit des trockenen Auges, die mit Autoimmunerkrankungen wie rheumatoider Arthritis, systemischem Lupus erythematodes und Morbus Gougerot-Sjögren in Verbindung stehen.

6. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach Anspruch 2 bei der Behandlung der Krankheit des trockenen Auges bei der Behandlung von allergischen Konjunktivitis mit verminderter Tränenfilmsekretion.

7. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung bei der Behandlung der Krankheit des trockenen Auges nach einem der Ansprüche 2 bis 6, **dadurch gekennzeichnet, dass** es in einer Dosis von 0,1 bis 5 mg/Tag verabreicht wird.

8. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach einem der Ansprüche 1 bis 7, **dadurch gekennzeichnet, dass** es in Kombination mit Feuchthaltemitteln auf Basis von Carbomeren oder Gleitmitteln auf Basis von Hyaluronsäure erfolgt.

9. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach einem der Ansprüche 1 bis 8, **dadurch gekennzeichnet, dass** es auf der Ebene mindestens einer seiner Methylgruppen durch deuterierte Methylgruppen substituiert ist.

10. Links- und rechtsdrehende Enantiomere von (3S)-6,7-Dimethoxy-3-[(5R)-4-methoxy-6-methyl-7,8-dihydro-5H-[1,3]dioxolo[4,5-g]-isochinolin-5-yl]-3H-2-benzofuran-1-on und pharmazeutisch unbedenkliche Salze davon zur Verwendung nach einem der Ansprüche 1 bis 9, **dadurch gekennzeichnet, dass** es in Form von Augentropfen oder Augensalben abgefüllt wird.

## Claims

1. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use in the treatment of diseases associated with decreased secretions of conjunctival epithelial cells, lacrimal glands and Meibomian glands.

2. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use in the treatment of diseases associated with ocular allergy.

3. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to claim 1 and 2 in the treatment of dry eye disease.

4. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to claim 2 in the treatment of allergic eye disease related to vernal keratoconjunctivitis.

5. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to claim 3 in the treatment of dry eye disease related to autoimmune diseases such as rheumatoid polyarthritis, systemic lupus erythematosus and Gougerot-Sjogren disease.

6. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to claim 2 in the treatment of dry eye disease in the treatment of allergic conjunctivitis with decreased tear film secretion.

7. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use in the treatment of dry eye disease according to one of claims 2 to 6, **characterised in that** it is administered at a dose of 0.1 to 5 mg/day.

8. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to one of claims 1 to 7, **characterised in that** it is in combination with carbomer-based humectants or hyaluronic acid-based lubricants.

9. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to one of claims 1 to 8, **characterised in that** it is substituted at least at one of its methyls with deuterated methyls.

10. The levorotatory and dextrorotatory enantiomers of (3S)-6,7-dimethoxy-3-[(5R)-4-methoxy-6-methyl-7,8-dihydro-5H-[1,3]dioxolo[4,5-g]isoquinolin-5-yl]-3H-2-benzofuran-1-one and its pharmaceutically-acceptable salts for use according to one of claims 1 to 9, **characterised in that** it is packaged in the form of eyedrops or ophthalmic ointment.
